# EUROPEAN PATENT APPLICATION

(11) **EP 1 835 285 A1**
(43) Date of publication of application: **19.09.2007**
(21) Application number: 06290407.3
(22) Date of filing: 14.03.2006
(51) Int. Cl.: G01N 33/574, C07K 16/18

(54) **ERCC1 expression in predicting response for cancer chemotherapy**

(71) Applicant: Institut Gustave Roussy, 94805 Villejuif Cedex (FR)
(72) Inventor: Fouret, Pierre, 75116 Paris (FR); Soria, Jean-Charles, 91430 Igny (FR)
(74) Representative: Fosse, Danièle

(57) **Abstract**

The present invention concerns an *in vitro* method for predicting the benefit of a postoperative cancer chemotherapy, said method comprising the step of the quantitative measurement of the ERCC1 protein by immunohistochemistry in a formalin-fixed paraffin-embedded sample from the patient's tumor.

## Description

### Technical field of the invention

The present invention is directed to the detection of the Excision Repair Cross-Complementation group 1 (ERCC1) enzyme by immunohistochemistry and its use in the prediction of the benefit of platinating agents-based cancer chemotherapy.

### Background art of the invention

Lung cancer is a leading cause of cancer deaths in most industrialized countries (Jemal A, Murray T, Ward E, et al. Cancer statistics, 2005. CA Cancer J Clin 2005;55:10-30). Despite complete tumor resection in patients with stage I-III non-small-cell lung cancer, distant metastases develop in 50-70 percent of patients.

Adjuvant chemotherapy has been tested to improve survival in patients with completely resected non-small-cell lung cancer. The recently reported International Adjuvant Lung Cancer Trial (IALT) with 1,867 patients, was designed to assess the potential benefit of adjuvant cisplatin-based chemotherapy after complete resection of non-small cell lung cancer. The IALT demonstrated a 4.1 percent absolute benefit in 5-year overall survival in non-small-cell lung cancer patients treated with adjuvant cisplatin-based chemotherapy (the International Adjuvant Lung Cancer Trial Collaborative Group. Cisplatin-based adjuvant chemotherapy in patients with completely resected non-small-cell lung cancer. N Engl J Med 2004;350:351-60). Several other randomized studies have confirmed the benefit of postoperative platinum-based therapy in non-small-cell lung cancer (Gurubhagavatula S, Lynch TJ. The role of adjuvant chemotherapy for non-small cell lung cancer. Semin Respir Crit Care Med 2005;26:298-303). However, adjuvant chemotherapy only slightly prolongs survival, with a 5-year overall survival improvement ranging from 4 to 15 percent, and gives rise to significant adverse effects (Winton T, Livingston R, Johnson D, et al. Vinorelbine plus cisplatin vs. observation in resected non-small-cell lung cancer. N Engl J Med 2005;352:2589-97). The identification and quantification of predictive factors for resistance or sensitivity to adjuvant cisplatin-based chemotherapy were therefore needed.

Among potential predictive factors are those involved in cisplatin resistance such as DNA repair mechanisms. Cisplatin induces cytotoxic effects by binding to DNA and creating platinum-DNA adducts. Some of these adducts establish covalent cross-linking between DNA strands, thereby inhibiting DNA replication. Among the DNA repair pathways, nucleotide excision repair plays a central role and has been associated with resistance to platinum-based chemotherapy (Reed E. Platinum-DNA adduct, nucleotide excision repair and platinum based anti-cancer chemotherapy. Cancer Treat Rev 1998;24:331-44). The excision repair cross-complementation group 1 (ERCC1) enzyme plays a rate-limiting role in the nucleotide excision repair pathway which recognizes and removes cisplatin-induced DNA adducts (Zamble DB, Mu D, Reardon JT, Sancar A, Lippard SJ. Repair of cisplatin--DNA adducts by the mammalian excision nuclease. Biochemistry 1996;35:10004-13). ERCC1 is also an important factor in DNA interstrand crosslink repair, as well as in recombination processes (De Silva IU, McHugh PJ, Clingen PH, Hartley JA. Defining the roles of nucleotide excision repair and recombination in the repair of DNA interstrand cross-links in mammalian cells. Mol Cell Biol 2000;20:7980-90).

For more than a decade, smaller studies have repeatedly reported an association between low *ERCC1* mRNA expression levels in several solid tumors and improved clinical outcomes in patients treated with platinum-containing regimens (Dabholkar M, Vionnet J, Bostick-Bruton F, Yu JJ, Reed E. Messenger RNA levels of XPAC and ERCC1 in ovarian cancer tissue correlate with response to platinum-based chemotherapy. J Clin Invest 1994;94:703-8). In particular, Lord *et al* (Lord RV, Brabender J, Gandara D, et al. Low ERCC1 expression correlates with prolonged survival after cisplatin plus gemcitabine chemotherapy in non-small cell lung cancer. Clin Cancer Res 2002;8:2286-91) reported that *ERCC1* mRNA expression predicts response to chemotherapy in advanced non-small-cell lung cancer. Furthermore, by using methodologies such as DNA isolation, enzymatic digestions, and DNA sequencing, two common polymorphisms of the *ERCC1* gene (codon 118 C/T and C8092A) were found to be correlated with response to platinum-based chemotherapy in colorectal (Viguier J, Boige V, Miquel C, et al. ERCC1 codon 118 polymorphism is a predictive factor for the tumor response to oxaliplatin/5-fluorouracil combination chemotherapy in patients with advanced colorectal cancer. Clin Cancer Res 2005;11:6212-7) and non-small-cell lung cancer (Zhou W, Gurubhagavatula S, Liu G, et al. Excision repair cross-complementation group 1 polymorphism predicts overall survival in advanced non-small cell lung cancer patients treated with platinum-based chemotherapy. Clin Cancer Res 2004;10:4939-43). These polymorphisms are mainly associated with lower translation rates of the *ERCC1* gene, resulting in low expression levels.

The invention described in the international application WO 02/061128 (published on 8^{th} August 2002) relates to prognostic methods for cisplatin-based cancer chemotherapy assessing ERCC1 expression levels. These prognostic tests consist of (i) determining a platinum-based chemotherapy by examination of the amount of ERCC1 mRNA in patient's tumor cells and (ii) comparing it to a pre-determined threshold expression level. Such quantitative gene expression studies were developed for formalin-fixed paraffin-embedded pathological samples because most tumor samples are routinely formalin-fixed paraffin-embedded to allow histological analysis and subsequent archival storage.

Nevertheless, (i) techniques for the isolation and analyses of mRNA from formalin-fixed paraffin-embedded tissue samples are frequently inaccurate, costly and time-consuming and (ii) the conservation of mRNA in formalin-fixed paraffin-embedded samples is eventually affected with time. For these reasons, such analyses are carried out with difficulty by the skilled person, especially in large-scale studies. Other techniques, allowing an easier, more accurate and less expensive measure of the expression of ERCC1 are thus particularly needed.

Moreover, since the study on which the international application WO 02/061128 (published on 8^{th} August 2002) was based did not compare two groups of patients according to whether or not they were treated with cisplatin, the value of *ERCC1* mRNA expression as evidenced in this study is only prognostic, and not predictive.

### Summary of the invention (Invention solution)

For the skilled person, as stated in the patent application WO 02/061128, protein expression levels can be only qualitatively monitored in formalin-fixed paraffin-embedded samples by using immunohistochemical methods. Surprisingly, the inventors have developed a quantitative and reproducible immunohistochemical method assessing ERCC1 gene expression levels which is the subject matter of the present invention.

Using said immunohistochemical method, the inventors have demonstrated, in a quantitative and reproducible way, that patients with ERCC1-negative tumors have a risk of death decreased by 33 % (hazard ratio 0.67) when cisplatin based chemotherapy is added to surgery. On the other hand, the inventors have found that the risk of death was not decreased by the adjunction of chemotherapy among patients with ERCC1-positive tumors (hazard ratio 1.18).

The analyses of the protein level by the said immunohistochemical method is predictive of survival in early-stage and completely resected non-small cell lung cancer. Because immunohistochemistry, in contrast with mRNA analyses, can be easily applied in every pathology laboratory, this invention is therefore widely applicable and a useful test in clinical practice.

This invention also presents an additonal advantage which is to be able to analyze formalin-fixed paraffin-embedded tumor samples, whatever the fixation techniques are.

Thus, the present invention provides an *in vitro* method for predicting the benefit of postoperative platinum-based cancer chemotherapy, said method comprising the step of the quantitative measurement of the ERCC1 protein by immunohistochemistry in a formalin-fixed paraffin-embedded sample from the patient's tumor.

In an "immunohistochemical method" a section of tissue is tested for specific proteins by exposing the tissue to antibodies that are specific for the protein that is being assayed. The antibodies are then visualized by any of a number of methods to determine the presence and amount of the protein present. Examples of methods used to visualize antibodies are, for example, through enzymes linked to the antibodies (e. g., luciferase, alkaline phosphatase, horseradish peroxidase, or P-galactosidase), or chemical methods (e.g., DAB/Substrate chromagen), gold, fluorescent or labelled antibodies by any of the many different methods known to those skilled in this art.

In embodiments of the said immunohistochemical method, detection or assaying the level of ERCC1 protein in a tumor sample includes contacting it with an antibody or antigen-binding fragments directed against ERCC1 protein or a fragment thereof; and determining the amount of the binding antibody on the tumor sample.

"Antibody" includes immunoglobulin molecules and the antigen binding fragments. The antibody can be a polyclonal antibody or a monoclonal antibody. The antibody can be labeled by a detectable means and includes enzymatically, radioactively, fluorescently, chemiluminescently or bioluminescently labeled antibodies by any of the many different methods known to those skilled in this art.

By "antigen-binding fragments" it is intended to encompassed particularly the fragments Fv, Fab, F(ab')2, Fab', scFv, scFv-Fc. These antibody fragments are obtained using conventional techniques well-known to those with skill in the art, and the fragments are screened for utility in the same manner as are intact antibodies.

In a preferred embodiment of the invention, the immunohistochemical method comprises the following steps :
(a) obtaining slides from formalin-fixed paraffin-embedded tumor samples
(b) retrieving epitope in buffer
(c) incubating slides with a monoclonal ERCC1 antibody
(d) determining the amount of binding antibodies on the formalin-fixed paraffin-embedded tumor samples, using the amount of binding antibodies on an internal positive control as a reference
(e) determining the percentage of labeled nuclei on the formalin-fixed paraffin-embedded tumor samples
(f) multiplying the value estimated in step (d) with the value estimated in step (e)
(g) determining a platinum-based chemotherapy regimen by comparing the value obtained in step (f) to a pre-determined threshold level.

With this method, steps (d) and (f) are used for the first time. They make the detection of ERCC1 surprisingly quantitative and reproducible.

In the method of the present invention, the anti ERCC1 antibody is preferably the mouse antibody clone 8F1 (NeoMarkers).

Is also preferred, a method according to the present invention, wherein the internal positive control consists of stroma cells surrounding the tumor area.

In a more preferred embodiment of the invention, such cancer is preferably a non-small-cell lung cancer.

It is also preferred that the cancer chemotherapy is based on cisplatin alone or associated with etoposide or a vinca alkaloid.

The invention also relates to a kit for the detection or quantification of human ERCC1 protein wherein said kit comprises antibodies and appropriate reagents and buffers.

### Description of the drawing

### Figure 1. Example of ERCC1 staining.

Figure 1A. Image (400X) of a strongly ERCC1-positive squamous cell carcinoma (Intensity =3).
Figure 1B. Image (400X) of an ERCC1-negative squamous cell carcinoma with positive internal controls (arrow).

### Figure 2. Kaplan-Meier estimates of the proportions of surviving patients.

Figure 2A. Overall survival according to treatment in all 761 patients. The adjusted hazard ratio for death in the chemotherapy group as compared with the control group was 0.87 (95 percent confidence interval, 0.71 to 1.06, P=0.17).
Figure 2B. Overall survival according to treatment in patients with ERCC1-negative tumors. The adjusted hazard ratio for death in the chemotherapy group as compared with the control group was 0.67 (95 percent confidence interval, 0.51 to 0.89, P<0.006).
Figure 2C. Disease-free survival according to treatment in patients with ERCC1-negative tumors. The hazard ratio for disease progression or death was 0.69 (95 percent confidence interval, 0.53 to 0.90, P<0.007).
Figure 2D. Overall survival according to treatment in patients with ERCC1-positive tumors. The adjusted hazard ratio for death in the chemotherapy group as compared with the control group was 1.18 (95 percent confidence interval, 0.87 to 1.61, P=0.29).

### Detailed description of the invention

### Example 1 : Materials and Methods.

### Patients and study design.

All patients had participated in the IALT study that compared adjuvant cisplatin-based chemotherapy to observation in patients with non-small-cell lung cancer. Inclusion criteria and the results of the IALT have already been reported (The International Adjuvant Lung Cancer Trial Collaborative Group. Cisplatin-based adjuvant chemotherapy in patients with completely resected non-small-cell lung cancer. N Engl J Med 2004;350:351-60), see table 1. Briefly, 1,867 patients with completely resected stage I-III non-small-cell lung cancer had been randomized to either chemotherapy with cisplatin (total dose 300-400 mg/m²) plus another drug (etoposide or a vinca alkaloid), or observation (control group). The median follow-up time was 56 months.

The IALT-Bio study was subsequently designed by a steering committee to examine whether immunohistochemically assessed tumor markers had the ability to predict a survival benefit from chemotherapy in formalin-fixed paraffin-embedded tumor samples collected from centers that had recruited more than 10 patients . To study whether the effect of chemotherapy was different between patients with a positive or a negative marker status, the estimated power to detect a 20 percent difference in the survival benefit at 5 years in 800 patients was 66 percent (two-sided, type I error 1 %). Twenty-eight centers in 14 countries (see table 1) contributed specimens. Approval was obtained from the local Institutional Review Boards according to the legal regulations in each participating country.

All tumors were reviewed centrally (Brambilla E, Lantuejoul S, Dunant A, et al. IALT - International Adjuvant Lung Cancer Trial - : Quality assessment and histopathological review according to the WHO 2004 classification and assessment of prognostic and predictive role of pathological criteria. Lung Cancer 2005;49 Suppl. 2:S44) according to the W.H.O. 2004 histopathological classification.

**TABLE 1: The IALT-Bio participating centers (investigators and pathologists)**

| | | |
|---|---|---|
| ***AUSTRIA:*** | • | *R. Pirker, Internal Medicine I, Vienna, G. Dekan, Institute of Pathology, Vienna* |
| ***BELGIUM:*** | • | *J. Vansteenkiste, University Hospital, Leuven* |
| **BRAZIL**: | • | I. Sathler Pinel, Instituto Nacional de Cancer, Rio de Janeiro |
| | • | R. Younes, Hospital A.C. Camarco, Sao Paulo |
| ***FRANCE:*** | • | *A.A. Kanoui, Centre Physiothérapie du Rouget, Sarcelles;* |
| | *R. Dachez, Laboratoire L.C.L., Paris;* | |
| | *S. Deslignères, Hospital Delafontaine, Saint-Denis;* | |
| | O. *Languille-Mimoune, Cabinet Pathologie, Paris;* | |
| | *P. Sabatier, Centre Hospitalier Victor Dupouy, Argenteuil* | |
| | • | *T. Le Chevalier, Institut Gustave-Roussy, Villejuif;* |
| | *M. Antoine, Hôpital Tenon, Paris* | |
| | *P. Boz, Cabinet de Pathologie, Papeete;* | |
| | *P. Bruneval, Association Promotion Anatomie Pathologique, Paris;* | |
| | *M.C. Charpentier, Cabinet Pathologie Tolbiac, Paris;* | |
| | *B. Chetaille, Hôpital Sainte Marguerite, Marseille;* | |
| | *E. Dulmet, Centre Chirurgical Marie-Lannelongue, Le Plessis Robinson;* | |
| | *F. Capron, Groupe Hospitalier Pitié-Salpétrière, Paris;* | |
| | *B. Gosselin, C.H.U., Lille;* | |
| | *D. Grunenwald, P. Validire, Institut Mutualiste Montsouris, Paris;* | |
| | *F. Labrousse, C.H.U., Limoges;* | |
| | *N. Pericoli, Roma (Italy);* | |
| | *D. Petrot, Cabinet d'Anatomie Pathologique, Niort;* | |
| | *N. Rouyer, Cabinet de Pathologie Butet-Rouyer, Nice* | |
| | • | *B. Milleron, M. Antoine, Hôpital Tenon, Paris* |
| | • | *J.F. Morère, M. Kambouchner, Hôpital Avicenne, Bobigny* |
| | • | *G. Ozenne, Ceditrac - CMC du Cèdre, Bois Guillaume* |
| | T. Ducastelle, Laboratoire d'Anatomie et Cytologie, Rouen | |
| | • | *E. Quoix, Hôpital Lyautey, Strasbourg;* |
| | *P. Durand de Grossouvre, Laboratoire d'Anatomie Pathologique, Haguenau;* | |
| | *B. Gasser, C.H.U., Strasbourg* | |
| | • | *A. Rivière, Centre François Baclesse, Caen;* |
| | *F. Galateau-Salle, CHU, Caen* | |
| | • | *C. Tuchais, P. Jallet, G. Bertrand, I. Valo, Centre Paul Papin, Angers* |
| ***GERMANY**:* | • | *W. Eberhardt, University Hospital, Essen;* |
| | *D. Theegarten, Institute of Pathology, Ruhr-University Bochum, Bochum* | |
| ***GREECE:*** | • | *P. Christaki, Papanikolaou General Hospital, Pylea* |
| | • | *T. Dosios, V. Kyriakou, Athens University School of Medicine, Athens* |
| | • | *E. Papadakis, P. Agelidou, Sotiria Hospital, Athens* |
| | • | *K. Zarogoulidis, University Hospital, Thessaloniki* |
| ***ITALY:*** | • | *A. Masotti, Azienda Ospedaliera Di Verona, Verona* |
| ***LITHUANIA:*** | • | *A. Jackevicius, Institute of Oncology Vilnius University, Vilnius* |
| ***POLAND:*** | • | *J. Laudanski, L.Chyczewski, M. Kozlowski, J. Niklinski, Medical School, Bialystok* |
| | • | *T. Grodski, J. Pankowski, Regional Hosp. For Lung Diseases, Szczecin* |
| | • | *T. Orlowski, M. Chabowski, R. Langfort, Institute of Tuberculosis and Lung Disease, Warsaw;* |
| | *B. Muszczynska-Bernhard, Dolnoslaskiego Centrum Chorob Pluc, Wroclaw* | |
| ***ROMANIA:*** | • | *T. Ciuleanu, Oncological Institute "Ion Chiricuta", Cluj-Napoca* |
| ***SLOVAKIA:*** | • | *J. Baumohl, University Teach. Hospital, Kosice* |
| ***SPAIN:*** | • | *F. Cardenal, Hospital Duran I Reynals, Barcelona; R. Bernat, Hospital de Bellvitge, Barcelona* |
| | • | *J. Salinas, J.B. Lopez, Hospital Virgen de Arrixaca, El Palmar Murcia* |
| ***SWEDEN:*** | • | *B. Bergman, A. Hussein, Sahlgrenska Hospital, Göteborg* |
| ***YUGOSLAVIA:** .* | • | *G. Radosavljevic, Institute for Lung Disease, Belgrade* |

### Immunostaining for ERCC1.

The epitopes were first retrieved in citrate buffer (10 mM, pH 6.0, heated for 30 minutes in a bain marie ), then slides were incubated at a 1:300 dilution over 60 minutes with the monoclonal ERCC1 mouse antibody (clone8F1, NeoMarkers, Fremont CA, USA) that was raised against the full-length human ERCC1 protein. Antibody binding was detected by means of an ABC-kit with NovaRED^{TM} as the substrate (Vectastain Elite, Vector Laboratories, Burlingame CA, USA) and Mayer's hematoxylin as the counterstain. Sections of normal tonsil tissues were included as external positive controls and stromal cells (endothelium) surrounding the tumor area served as internal positive controls.

### Microscopic analysis

Two investigators who where blinded to clinical data, independently evaluated ERCC1 staining under the light microscope at x400 magnification. We recorded whether or not tumor or stromal cells expressed ERCC1. In addition, staining intensity was graded on a scale of 0 to 3 (using endothelial cells in tonsil controls as a reference point [intensity 2]). Discordant cases were reviewed. Cases without valid internal controls were excluded. Five images of representative areas were acquired at x400 magnification for each case. All positive or negative tumor nuclei (a total of 500-1,500 tumor nuclei per case) were manually counted on a computer screen using ImageJ freeware edited by the National Institutes of Health (http://rsb.info.nih.gov/ij). The percentage of positive tumor nuclei was calculated per case and a proportion score was attributed (0 if 0 percent; 0.1 if 1 to 9 percent; 0.5 if 10 to 49 percent; 1.0 if 50 percent or more), as previously described (Al Haddad S, Zhang Z, Leygue E, et al. Psoriasin (S100A7) expression and invasive breast cancer. Am J Pathol 1999;155:2057-66 or Handra-Luca A, Bilal H, Bertrand JC, Fouret P. Extra-cellular signal-regulated ERK-1/ERK-2 pathway activation in human salivary gland mucoepidermoid carcinoma: association to aggressive tumor behavior and tumor cell proliferation. Am J Pathol 2003;163:957-67). In each case, the proportion score was multiplied by the staining intensity of nuclei to obtain a final quantitative H-score (among 9 possible ones). The median value of the H-scores was *a priori* chosen as the cut-off point for separating ERCC1-positive from ERCC1-negative tumors.

### Statistical analysis.

As in the IALT, the primary endpoint was overall survival after the date of randomization. Disease-free survival was analyzed as a secondary endpoint. In order to study selection bias within the IALT-Bio participating centers, the pre-randomization characteristics and overall survival of the two groups of patients (with or without blocks) were compared using a Cox model. Baseline data according to the ERCC1 status were compared in univariate_analyses with Chi-square tests and with a multivariate logistic model.

Survival rates were estimated using the Kaplan-Meier method. The predictive values of the ERCC1 status and chemotherapy for survival were studied using the Cox model. As in the IALT analysis, the Cox model included every factor used in the stratified randomization (center, disease stage, and type of surgery), plus clinical and histological predictive factors (age, sex, W.H.O. performance status, and revised histopathological type). All other factors that were statistically related to the ERCC1 status in the multivariate logistic model (P<0.05) were added to the survival Cox model (pathological T status, and pleural invasion). The predictive value of ERCC1 was studied by testing the interaction between the ERCC1 status and the attributed treatment (chemotherapy or no chemotherapy) in the same Cox model. All reported P values were two-sided. P values below 0.01 were considered statistically significant in order to limit the risk of false positive results. All analyses were performed using SAS software, version 8.2.

### Examples 2 : Patient characteristics

The 28 centers which participated in the IALT-Bio study included 1045 patients in the original IALT study. They were able to provide one tumor block for only 867 patients (83 percent). These 867 patients were comparable to the remaining 178 in terms of pre-randomization characteristics and overall survival. The amount and quality of the 824 blocks were adequate for serial sectioning. Among these blocks, 783 contained tumor material corresponding to non-small-cell lung cancer and were included in the IALT-Bio study. After exclusion of cases without valid positive internal controls, ERCC1 expression was evaluated in 761 cases. All further statistical analyses were based on these 761 patients.

The characteristics of the IALT-Bio study patient population are summarized in Table 1. A total of 426 cases were squamous-cell carcinomas (56 percent), 242 adenocarcinomas (32 percent), and 93 were of another histological type (12 percent). Median age was 58 years (range 27-77) and the great majority were males (81.6 percent). Three hundred and eighty-nine patients (51 percent) were randomized to receive adjuvant cisplatin-based chemotherapy, whereas 372 (49 percent) were randomized to the control group.

### Example 3 :Immunohistochemically assessed ERCC1 expression

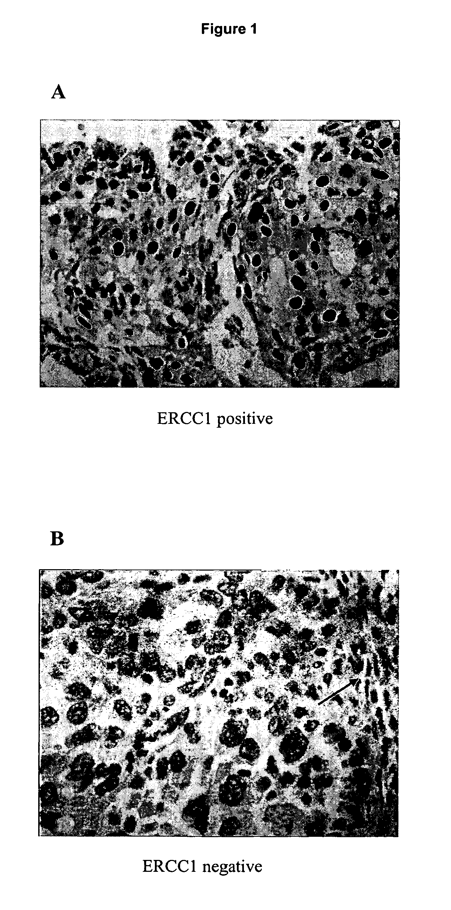

As illustrated in Figure 1, ERCC1 immunostaining was nuclear. The median value of the percentage of stained cells was 24 percent (range 0 to 100 percent), whereas the median value of H-scores was 1.0. Tumors with an H-score exceeding 1.0 (i.e. tumors with a staining intensity score of 2 and 50 percent or more positive nuclei or a staining intensity score of 3 and 10 percent or more positive nuclei) were deemed ERCC1 positive, which was the case in 335 patients (44 percent). The median H-score alone (1.0) was attributed to 164 tumors (22 percent). The main differences in clinico-pathological parameters according to ERCC1 expression are reported in Table 2 (univariate analysis). Using the multivariate logistic model, ERCC1 expression was significantly correlated with age (P=0.02 lower in young patients), sex (P=0.04 lower in females), pathological T status (P=0.04 lower with a higher T status), histological type (lower in adenocarcinomas P<0.0001), and pleural invasion (P=0.01 higher in the case of pleural invasion).

### Example 4 : Overall survival and ERCC1 expression

The 5-year overall survival rate was 43 percent, 95 percent confidence interval [39 to 47 percent] (Table 3) for the total study-population. Using the Cox model, ERCC1 expression had no predictive value for the entire study population (adjusted hazard ratio for death, 0.87; 95 percent confidence interval [0.69 to 1.09], P=0.23).

### Example 5 : Overall survival and adjuvant chemotherapy

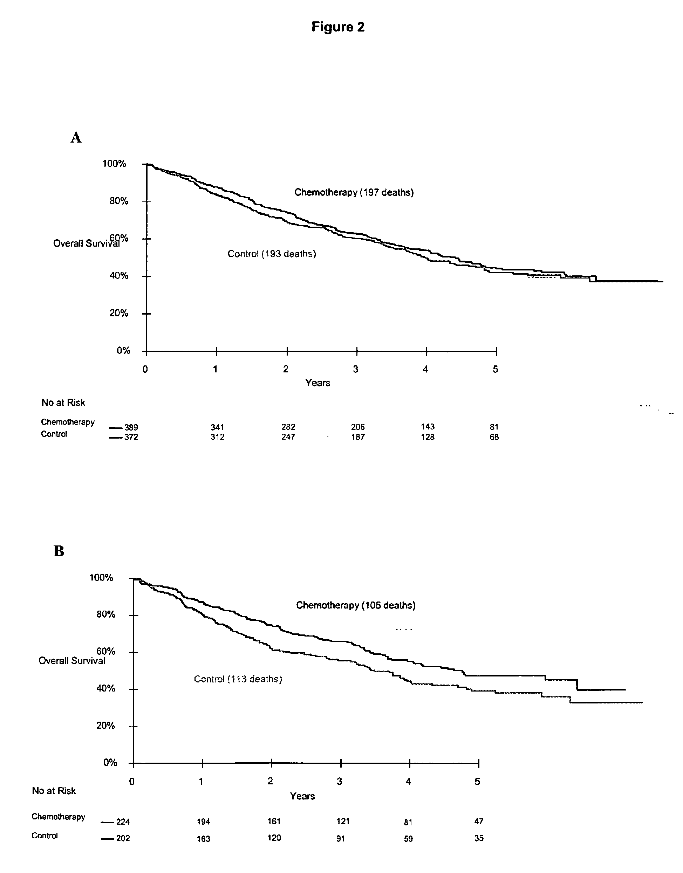

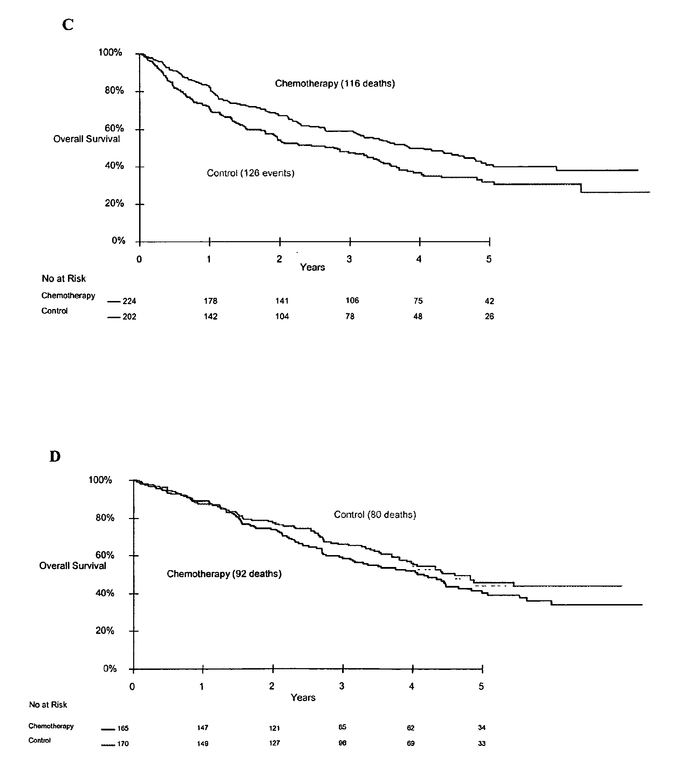

The 5-year overall survival rates were 44 percent (95 percent confidence interval [39 to 50 percent]) and 42 percent (95 percent confidence interval [37 to 48 percent]) in the chemotherapy group and control group respectively (Table 3). In the Cox model, the adjusted hazard ratio for death was 0.87 (95 percent confidence interval [0.71 to 1.06], P=0.17) in favor of chemotherapy (Table 3, Figure 2A).

### Example 6 : Benefit of adjuvant chemotherapy according to ERCC1 expression.

The interaction term between ERCC1 expression and treatment was statistically significant (for overall survival, P<0.009). In patients with ERCC1-negative tumors, overall survival was significantly higher in the chemotherapy group compared to the control group (adjusted hazard ratio for death, 0.67; 95 percent confidence interval [0.51 to 0.89] P<0.006) (Table 3). The 5-year survival rates were 47 percent (95 percent confidence interval [40 to 55 percent]) and 39 percent (95 percent confidence interval [32 to 47 percent]) respectively. Median overall survival was 14 months longer in the adjuvant chemotherapy group compared to the control group of patients with ERCC1-negative tumors (56 and 42 months respectively, Figure 2B). Disease-free survival in patients with ERCC1-negative tumors was also significantly higher in the chemotherapy group compared to patients randomized to observation (adjusted hazard ratio for recurrence or death, 0.69; 95 percent confidence interval [0.53 to 0.90], P<0.007) (Figure 2C).

There was no survival difference between the adjuvant chemotherapy group and the control group among patients with ERCC1-positive tumors (adjusted hazard ratio for death, 1.18; 95 percent confidence interval [0.87 to 1.61], P=0.29) (Table 3, Figure 2D).

When the analysis focused exclusively on patients in the control group, the 5-year overall survival rate was significantly higher in patients with ERCC1-positive tumors (46 percent, 95 percent confidence interval [37 to 55 percent]) than in patients with ERCC1-negative tumors (39 percent, 95 percent confidence interval [32 to 47 percent]), with an adjusted hazard ratio of 0.65, 95 percent confidence interval [0.48 to 0.89], P<0.008 (Table 3).

**TABLE 2. Patient Characteristics**

| **Characteristic** | **Total** N=761 (percent) | **ERCC1+** N=335 (percent) | **ERCC1 -**N=426 (percent) | **P-value^{*}** |
|---|---|---|---|---|
| Age | | | | P<0.003 |
| <55 yr | 231 (30) | 80 (24) | 151 (35) | (P for trend: |
| 55-64 yr | 330(43) | 161 (48) | 169(40) | P<0.008) |
| >64 yr | 200 (26) | 94 (28) | 106 (25) | |
| Sex | | | | P<0.0005 |
| Male | 621 (82) | 292 (87) | 329 (77) | |
| Female | 140(18) | 43(13) | 97(23) | |
| Pathological TNM stage | | | | P=0.97 |
| Stage I | 267 (35) | 119 (35) | 148 (36) | |
| Stage II | 175 (23) | 76 (23) | 99 (23) | |
| Stage III | 319 (42) | 140 (42) | 179 (42) | |
| T of TNM | | | | P=0.10 |
| 1 | 118 (16) | 60(18) | 58(14) | |
| 2 | 452 (59) | 188 (56) | 264 (62) | |
| 3 | 181 (24) | 85 (25) | 96 (23) | |
| 4 | 10(1) | 2 (1) | 8(2) | |
| Histological type | | | | P<0.0001 |
| Squamous cell carcinoma | 426 (56) | 236 (70) | 190 (45) | |
| Adenocarcinoma | 242 (32) | 71 (21) | 171 (40) | |
| Other | 93(12) | 28(8) | 65(15) | |
| Performance Status | | | | P=0.06 |
| 0 | 426 (56) | 188 (56) | 238 (56) | |
| 1 | 276(36) | 113 (34) | 163(38) | |
| 2 | 59 (8) | 34 (10) | 25 (6) | |
| Pleural invasion | | | | P<0.007 |
| Yes | 61 (8) | 37(11) | 24 (6) | |
| No | 700 (92) | 298 (89) | 402 (94) | |
| Vascular invasion | | | | P=0.04 |
| Yes | 222 (29) | 85 (25) | 137 (32) | |
| No | 539(71) | 250(75) | 289(68) | |
| Surgery | | | | P=0.35 |
| Pneumonectomy | 306 (40) | 141 (42) | 165 (39) | |
| Segment-/lobectomy | 455 (60) | 194 (58) | 261 (61) | |
| Radiotherapy | | | | P=0.35 |
| Yes | 199 (26) | 82 (24) | 117 (27) | |
| No | 562 (74) | 253 (76) | 309 (73) | |
| Planned cisplatin dose | | | | P=0.67 |
| 80 mg/m² per cycle | 139 (18) | 58 (17) | 81 (19) | |
| 100 mg/m² per cycle | 544 (71) | 245 (73) | 299 (70) | |
| 120 mg/m² per cycle | 78 (10) | 32 (10) | 46(11) | |

| | | | | |
|---|---|---|---|---|
| *: Chi-square, univariate analysis | | | | |

**TABLE 3 Variation of overall survival according to attributed treatment and ERCC1 status**

| | **Chemotherapy** | **Control group** | **All patients** | **Adjusted Hazard ratio for death (chemotherapy vs. controls)** [95% Cl] |
|---|---|---|---|---|
| **ERCC1-negative tumors** | 105 / 224 | 113/202 | 218 / 426 | 0.67 |
| | 47% [40%-55%] | 39% [32%-47%] | 44% [38%-49%] | [0.51-0.89] |
| | 56 months | 42 months | 48 months | P<0.006 |
| **ERCC1-positive tumors** | 92 / 165 | 80 / 170 | 172 / 335 | 1.18 |
| | 40% [32%-49%] | 46% [37%-55%] | 43% [37%-49%] | [0.87-1.61] |
| | 50 months | 55 months | 52 months | P=0.29 |
| **All patients** | 197 / 389 | 193 / 372 | 390 / 761 | 0.87 |
| | 44% [39%-50%] | 42% [37%-48%] | 43% [39%-47%] | [0.71-1.06] |
| | 53 months | 48 months | 50 months | P=0.17 |
| **Adjusted Hazard ratio for death (ERCC1 positive vs. ERCC1 negative)** [95% Cl] | 1.15 | 0.65 | 0.87 | **Test for interaction ERCC1*treatment P<0.009** |
| | [0.85-1.56] | [0.48-0.89] | [0.69-1.09] | |
| | P=0.38 | P<0.008 | P=0.23 | |

| | | | | |
|---|---|---|---|---|
| Cl denotes confidence interval - the central grey □ cells contain the following information : number of deaths, number of patients, 5-year survival rate and the 95 percent confidence interval, and median survival. | | | | |

## Claims

1. An *in vitro* method for predicting the benefit of a postoperative cancer chemotherapy, said method comprising the step of the quantitative measurement of the ERCC1 protein by immunohistochemistry in a formalin-fixed paraffin-embedded sample from the patient's tumor.

2. An *in vitro* method according to claim 1 comprising the steps of :
(a) obtaining slides from formalin-fixed paraffin-embedded tumor samples
(b) retrieving epitope in buffer
(c) incubating slides with a monoclonal ERCC1 antibody
(d) determining the amount of binding antibodies on the formalin-fixed paraffin-embedded tumor samples, using the amount of binding antibodies on an internal positive control as a reference
(e) determining the percentage of labeled nuclei on the formalin-fixed paraffin-embedded tumor samples
(f) multiplying the value estimated in step (d) with the value estimated in step (e)
(g) determining a platinum-based chemotherapy regimen by comparing the value obtained in step (f) to the median score of the values obtained in step (f).

3. The method according to claim 1 to 2 wherein the anti ERCC1 antibody is the mouse antibody clone8F1 (NeoMarkers).

4. The method according to claim 1 to 3 wherein the internal positive control consists of stroma cells surrounding the tumor area.

5. The method according to claim 1 to 4 wherein said cancer is a non-small-cell lung cancer.

6. The method according to claim 1 to 5 wherein said cancer chemotherapy is a platinum-based cancer chemotherapy.

7. The method according to claim 1 to 6 wherein said cancer chemotherapy is based on cisplatin.

8. The method according to claim 1 to 7 wherein said cancer chemotherapy is a based on cisplatin associated with other chemotherapeutic agents.

9. The method according to claim 1 to 8 wherein said cancer chemotherapy is based on cisplatin with etoposide or a vinca alkaloid.

10. Kit for the detection of human ERCC1 protein wherein said kit comprises antibodies and appropriate reagents and buffers.

11. Kit according to claim 10 wherein said antibody is the monoclonal ERCC1 mouse antibody clone8F1, commercialized by Neomarkers.
